# EUROPÄISCHE PATENTANMELDUNG

(11) **EP 2 497 479 A1**
(43) Veröffentlichungstag der Anmeldung: **12.09.2012**
(21) Anmeldenummer: 11157187.3
(22) Anmeldetag: 07.03.2011
(51) Int. Cl.: A61K 35/66, A61K 39/00

(54) **Extrakte aus phototrophen Mikroorganismen als Adjuvans**

(71) Anmelder: IGV Institut für Getreideverarbeitung GmbH, 14558 Nuthetal (DE); RIPAC-LABOR GmbH, 14476 Potsdam (DE)
(72) Erfinder: Die Erfindernennung liegt noch nicht vor
(74) Vertreter: Hoppe, Georg Johannes

(57) **Zusammenfassung**

Die Erfindung betrifft eine Zusammensetzung (Vakzine), aufweisend einen Extrakt aus einem phototrophen Mikroorganismus als Adjuvans und einen Immunstimulator (Antigen) zur Generierung einer aktiven Immunität gegen eine Infektion bei einem Tier oder Mensch.

## Beschreibung

Die Erfindung betrifft die Verwendung eines Extrakts aus einem phototrophen Mikroorganismus in der Medizin, insbesondere als Adjuvans für Impfstoffe.

Bei der Aufzucht von Tieren verursachen Mikroorganismen häufig Erkrankungen, die zu Tierverlusten, Minderung der Wachstums- und Reproduktionsraten und zu wirtschaftlichen Einbußen führen können. Zur Therapie werden Antibiotika eingesetzt, deren Verwendung wegen der wachsenden Gefahr der Resistenzausbildung und ihrer Nebenwirkungen auf Umwelt und Mensch zunehmend stärker reglementiert wird.

Eine Alternative ist die Gesunderhaltung der Tiere mit Hilfe prophylaktischer Schutzimpfungen gegen relevante Krankheitserreger.

Die Impfstoffe (Vakzine) werden aus den abgetöteten oder abgeschwächten Krankheitserregern oder deren Bestandteilen hergestellt. Ihre Verabreichung verursacht eine komplexe erregerspezifische Immunantwort, die von vielen Faktoren beeinflusst wird.

Im Stand der Technik ist bekannt, dass die Wirkung von Impfstoffen durch Adjuvantien verstärkt werden kann. Bekannt Adjuvantien sind Aluminiumhydroxid und Mineralöl, die jedoch häufig unerwünschte Effekte verursachen. Daher besteht ein Bedarf an alternativen Adjuvantien.

### Beschreibung

Die vorliegende Erfindung betrifft immunstimulierende Extrakte aus phototrophen Mikroorganismen, die die Effizienz der Immunantwort eines Wirts auf einen Immunstimulator (z. B. ein Antigen) zur Generierung einer aktiven Immunität verbessern. Die Extrakte können als Adjuvans den Antigenen zugesetzt und mit den Vakzinen appliziert werden.

Unter einem Adjuvans ist hierbei eine Substanz zu verstehen, die als Mischung mit einer Substanz (Antigen) eine Immunantwort auslöst und die Bildung von Antikörpern induziert, oder getrennt davon appliziert wird und in unspezifischer Weise die Immunantwort auf dieses Antigen verstärkt oder verändert.

In einem ersten Aspekt betrifft die Erfindung die Verwendung eines Extrakts aus einem phototrophen Mikroorganismus in der Medizin, insbesondere als Adjuvans für die Impfung.

Der hier verwendete Begriff "phototropher Mikroorganismus" bezieht sich insbesondere auf Algen und Cyanobakterien. Die phototrophen Mikroorganismen können ausgewählt sein aus der Gruppe der Chlorophyta, Rhodophyta, Heterokontophyta und Cyanobacteria.

Der hier beschriebene Extrakt, also der Auszug oder die Lösung von Wirkstoffen aus den phototrophen Mikroorganismen, ist bevorzugt ein wässriger oder wässrig-ethanolischer Extrakt.

In einem zweiten Aspekt betrifft die Erfindung eine (pharmazeutische) Zusammensetzung (also eine Präparation, die eine Vakzine dargestellt), die als Komponenten mindestens einen Extrakt aus einem phototrophen Mikroorganismus und einen Immunstimulator zur Generierung einer aktiven Immunität aufweist.

In einer Ausführungsform der Erfindung kann die Zusammensetzung auch mindestens ein weiteres Adjuvans aufweisen. Das weitere Adjuvans kann insbesondere Öl, Aluminiumhydroxid, komplettes Freund'sches Adjuvans, inkomplettes Freund'sches Adjuvans, stabilisierende kationische Peptide ausgewählt aus Polypeptiden, Protamin, Nukleolin, Spermin oder Spermidin, kationischen Polysacchariden, Chitosan, TDM, MDP, Muramyldipeptid, oder Alaun-Lösung, Pluronics und Lipopeptide, einschließlich Pam3Cys sein, die allesamt dem Fachmann bekannt sind. Wie eingangs erwähnt geht die Verwendung derartiger Adjuvantien jedoch häufig mit unerwünschten Nebenwirkungen einher, bspw. sehr schmerzhaften Reizungen und Entzündungen am Ort der Applikation des immunisierten Tieres oder Menschen.

Die Zusammensetzung kann optional einen pharmazeutisch geeigneten Träger und/oder weitere Hilfs- und Zusatzstoffe aufweisen.

Der hier verwendete Begriff "pharmazeutisch geeigneter Träger" umfasst bevorzugt einen oder mehrere kompatible feste oder flüssige Füllstoffe, bzw. Verdünnungsmittel oder einkapselnde Verbindungen, welche für die Verabreichung geeignet sind. Die pharmazeutisch geeigneten Träger müssen selbstverständlich eine ausreichend hohe Reinheit und eine ausreichend geringe Toxizität aufweisen, um sie für die Verabreichung an eine zu behandelnde Person oder ein zu behandelndes Tier geeignet zu machen. Einige Beispiele von Verbindungen, welche als pharmazeutisch geeignete Träger oder Bestandteile davon dienen können, sind Zucker, wie beispielsweise Lactose, Glucose und Sukrose; Stärken wie beispielsweise Kornstärke oder Kartoffelstärke; Cellulose und seine Derivate, wie beispielsweise Natriumcarboxymethylcellulose, Ethylcellulose, Celluloseacetat; pulverisiertes Tragacanth; Malz; Gelatine; Talg; feste Gleitmittel, wie beispielsweise Stearinsäure, Magnesiumstearat; Kalziumsulfat; vegetabile Öle, wie beispielsweise Erdnussöl, Baumwollsamenöl, Sesamöl, Olivenöl, Kornöl und Öl aus Theobroma; Polyole, wie beispielsweise Polypropylenglycol, Glycerin, Sorbitol, Mannitol und Polyethylenglycol; Algininsäure; Emulgatoren, wie beispielsweise Tween^{®}; Benetzungsmittel, wie beispielsweise Natriumlaurylsulfat; färbende Agenzien; geschmacksvermittelnde Agenzien, Arzneistoffträger; tablettenbildende Agenzien; Stabilisatoren; Antioxidantien; Konservierungsmittel; pyrogen-freies Wasser; isotonische Salzlösung und phosphatgepufferte Lösungen.

Die Wahl eines pharmazeutisch geeigneten Trägers wird grundsätzlich durch die Art bestimmt, durch die die erfindungsgemäßen pharmazeutischen Zusammensetzungen verabreicht werden. Die erfindungsgemäßen pharmazeutischen Zusammensetzungen können bspw. systemisch verabreicht werden. Routen zur Verabreichung schließen z.B. transdermale, orale, parenterale, einschließlich subkutane oder intravenöse Injektionen, topische und/oder intranasale Routen ein. Die geeignete Menge der zu verwendenden pharmazeutischen Zusammensetzung kann durch Routineexperimente mit Tiermodellen (z. B. Kaninchen, Schaf, Maus, Ratte, Hund und nicht-humane Primatenmodelle) bestimmt werden. Bevorzugte Einheitsdosisformen zur Injektion schließen sterile Lösungen von Wasser, physiologischer Salzlösung oder Mischungen davon mit ein. Geeignete Träger zur Injektion schließen Hydrogele, Vorrichtungen zur kontrollierten oder verzögerten Freigabe, polylaktische Säure und Collagenmatrizen mit ein. Pharmazeutisch geeignete Träger zur topischen Anwendung schließen solche mit ein, die zur Verwendung in Lotionen, Cremes, Gels u. ä. geeignet sind. Falls die Verbindung oral verabreicht werden soll, sind Tabletten, Kapseln u. ä. die bevorzugte Einheitsdosisform. Die pharmazeutisch geeigneten Träger zur Herstellung von Einheitsdosisformen, die für die orale Verabreichung verwendbar sind, sind im Stand der Technik bekannt. Ihre Auswahl wird von sekundären Überlegungen wie Geschmack, Kosten und Lagerfähigkeit abhängen, welche für die Zwecke der vorliegenden Erfindung nicht kritisch sind, und ohne Schwierigkeiten durch einen Fachmann durchgeführt werden können.

Die Zusammensetzung gemäß der vorliegenden Erfindung umfasst typischerweise eine sichere und effektive Menge des erfindungsgemäßen immunstimulierenden Adjuvans. Eine "sichere und effektive Menge" ist eine solche Menge einer Verbindung, die ausreichend ist, eine effektive Immunantwort zur Bekämpfung einer Infektionserkrankung zu stimulieren. Gleichzeitig ist eine "sichere und effektive Menge" jedoch gering genug, um schwerwiegende Nebeneffekte zu vermeiden, also ein vernünftiges Verhältnis von Vorteil und Risiko zu ermöglichen. In Bezug auf das erfindungsgemäße immunstimulierende Adjuvans bedeutet der Begriff "sichere und effektive Menge" bevorzugt eine solche Menge, die geeignet ist, das Immunsystem in einer solchen Weise zu stimulieren, dass keine überschießenden bzw. schädlichen Immunreaktionen erzielt werden, jedoch bevorzugt auch keine solchen Immunreaktionen unterhalb eines messbaren Niveaus. Eine "sichere und effektive Menge" eines erfindungsgemäßen Adjuvans wird im Zusammenhang mit dem besonderen zu behandelnden Zustand variieren, sowie dem Alter und dem physischen Zustand des zu behandelnden Patienten, der Dauer der Behandlung, der Natur der begleitenden Maßnahmen, des verwendeten besonderen pharmazeutisch geeigneten Trägers und ähnlichen Faktoren innerhalb des Wissens und der Erfahrung des begleitenden Arztes entsprechen. Die erfindungsgemäße Zusammensetzung kann für humane und wie auch für veterinärmedizinische Zwecke eingesetzt werden.

In einem dritten Aspekt betrifft die Erfindung die Verwendung einer Zusammensetzung der hier beschriebenen Art in der Medizin, insbesondere im Rahmen der Impfung als Adjuvans.

In einem vierten Aspekt betrifft die Erfindung ein Verfahren zur Herstellung einer Zusammensetzung nach Anspruch 1 bis 4, das die folgenden Schritte aufweist:
- Bereitstellen eines phototrophen Mikroorganismus,
- Herstellen eines Extraktes aus dem phototrophen Mikroorganismus und
- Mischen des Extrakts aus dem phototrophen Mikroorganismus mit einem Immunstimulator (z B. einem Antigen) zur Generierung einer aktiven Immunität.

Der Immunstimulator zur Generierung einer aktiven Immunität kann ein Peptid, Protein, Zucker und/oder Lipopolysaccharid sein.

In einem fünften Aspekt betrifft die Erfindung ein Verfahren zur Immunisierung eines Tieres gegen eine Infektionskrankheit. Erfindungsgemäß weist das Verfahren den Schritt der Verabreichung einer hierin beschriebenen Zusammensetzung auf.

Die Verabreichung kann gemäß jeder im Stand der Technik bekannten Applikationsart erfolgen, z. B. durch transdermale, orale, parenterale, einschließlich subkutane oder intravenöse Injektionen, topische und/oder intranasale Routen. Bevorzugt ist die subkutane Applikation.

Das bei dem Immunisierungsverfahren verwendete Tier ist in einer Ausführungsform der Erfindung ein Wirbeltier, bevorzugt ein Säugetier oder ein Vogel oder Fisch. Als Säugetiere sind Nutz- bzw. Haustiere oder Menschen bevorzugt. Das Haustier kann beispielsweise ein Rind, Schwein, Pferd, Esel, Kamel, Katze, Hund Kaninchen, Ren, Huhn, Pute, Gans, Ente, das Nutztier ein Pelztier, z. B. Nerz, Nutia, oder ein Fisch sein. Das zu impfende Tier kann auch ein Wildtier oder ein gezüchtetes bzw. in Gefangenschaft lebendes Tier (Zootier) wie beispielsweise ein Strauß, Lama oder Damwild sein.

Mit dem Immunisierungsverfahren kann eine Immunität sowohl gegen eine virale, als auch gegen eine bakterielle Infektionskrankheit erreicht werden. Die bakterielle Infektionskrankheit kann durch gram-negative oder durch gram-positive Erreger verursachbar sein.

Mit dem hier beschriebenen Extrakt kann durch Zumischung zu einem Antigen die Schutzwirkung der Impfstoffe praktisch gegen jede virale oder bakterielle Infektionskrankheit der Wirbeltiere verbessert werden, beispielsweise gegen die folgenden Infektionskrankheiten bzw. Erreger:
Influenza, SARS, Gelbfieber, AIDS, Condyloma acuminata, Dellwarze, Dengue-Fieber, Dreitagefieber, Ebolavirus, Frühsommermeningoenzephalitis (FSME), Gürtelrose, Hepatitis, Herpes-Simplex Typ I, Herpes-Simplex Typ II, Herpes zoster, Japanische Enzephalitis, Lassafieber, Marburg-Virus, Masern, Maul- und Klausenseuche, Mononukleose, Mumps, Norwalk-Virus-infektion, Pfeifersches-Drüsenfieber, Pocken, Polio (Kinderlähmung), Pseudokrupp, Ringelröteln, Tollwut, Warzen, West-Nil-Fieber, Windpocken, Zytomegalie-Virus (CMV), bakteriellen Infektionskrankheiten wie Lyme-Borreliose, bakterielle Meningitis, Anthrax, Appendizitis (Blinddarmentzündung), Botulismus, Campylobacter, Chlamydia trachomatis (Harnröhren-, Bindehautentzündung), Cholera, Diphtherie, Donavanosis, Epiglottitis, Fleckfieber, Flecktyphys, Gasbrand, Gonorhoe, Tularämie, Heliobacter pylori, Bordetella pertussis, klimatischer Bubo, Knochenmarksentzündung, Legionärskrankheit, Lepra, Listeriose, Mittelohrentzündung, Mycoplasma hominis, Neugeborenensepsis, Noma, Paratyphus, Pest, Reitersyndrom, Rocky Mountain spotted fever, Salmonellen-Paratyphus, Salmonellen-Typhus, Scharlach, Syphilis, Tetanus, Tripper, Tuberkulose, Typhus, Vaginitis (Kolpitis), Weicher Schanker; sowie E. coli-Infektionen und Intoxikationen (E. coli-Ruhr, E.coli-Enterotoxämie, E. coli-Septikämie), Salmonelleninfektionen )insbesondere S. cholera suis, S. dublin, S. gallinarum, S. pullorum, S. enteritis, S. thyphimurium), Streptokokkeninfektionen (Druse, Streptokokken-Infektionen beim Schwein, Sudden death-Syndrom bei Enten, Streptococcus zooepidemicuns-Infektion), Staphylokokkeninfektionen (Staph. hyicus-, Staph. aureus-, Staph. intermedius-Infektionen), Pasteurellen-Infektionen (Pasteurella multocida-Infektionen, Geflügelcholera, haemorrhagische Septicaemia, Wild-und Rinderseuche), Rhinitis atrophicans, Mannheimia-haemolytica-Infektion, Moraxella-Infektionen (infektiöse Keratoconjunctivitis infolge von Moraxella bovis-Infektion u. a.), Erysipelothrix rhusiopathiae-Infektion (Rotlauf, Septikämie beim Geflügel und bei Nagetieren), Moderhinke der Schafe (Bacteroides nodosus-Infektion, Fusobacterium necrophoruminfektion), Nekrobazillose der Tiere und des Menschen (Fusobacterium necrophorumilnfektion), Clostridien-Infektionen und -intoxikationen (Rauschbrand, Pararauschbrand, Botulismus, Tetanus, CI. perfringens-Enterotoxämie, Gasbrand, ulzerative Enteritis der Vögel, Nekrotische Hepatitis, Bradsot, CI. Spiriformes- Infektionen, Malignes Ödem, Nekrotisierende Mastitis, Bazilläre Hämoglobinurie), Bordetellose (Bordetella bronchiseptica-Infektion, Bordetella avium-Infektion), Riemerellose (R. anatipestifer-Infektion, R. columbina-Infektion), Hämophilose (Glässersche Krankheit, Haemophilus gallinarum-Infektion), Actinobacillus-pleuropneumoniae-Infektion der Schweine, Actinobacillus equuli-Infektion der Pferde, Chlamydien-Infektionen (Enzootischer Abort der Schafe, Chlamydieninfektionen der Rinder), Ornithobacterium rhinotracheale- Infektion, Mycoplasmen-Infektionen, Arcanobacterium pyogenes-Infektion, bakterielle Infektionen bei Fischen (Furunkulose/Aeromonas salmonicida-Infektion, Rotmaulkrankheit / Yersinia ruckeri-Infektion, Vibriosen (Vibrio anguillarum-Infektion).

Die erfindungsgemäße Zusammensetzung (Vakzine) kann einen Anteil des Extrakts aus einem phototrophen Mikroorganismus von 1 % bis 60 % (Gew.%) als Adjuvans enthalten, bevorzugt 5% bis 30 %, besonders 10 % bis 20 %. Weiterhin kann das Vakzin ein herkömmliches Adjuvans enthalten, wie Öl oder Aluminiumhydroxid. Das herkömmliche Adjuvans kann insbesondere einen Anteil von 1 % bis 50 % (Gew.%) an der Vakzine ausmachen.

In einem sechsten Aspekt betrifft die Erfindung ein Adjuvans, das durch Bereitstellen eines phototrophen Mikroorganismus und Herstellen eines wässrigen Extraktes aus dem phototrophen Mikroorganismus herstellbar ist. Bevorzugte Ausgestaltungen dieses Erfindungsaspekts ergeben sich aus der hier angegebenen Offenbarung.

In einem siebenten Aspekt betrifft die Erfindung einen Kit, der ein wässriges Extrakt aus einem phototrophen Mikroorganismus und einen Immunstimulator zur Generierung einer aktiven Immunität aufweist.

In einem achten Aspekt betrifft die Erfindung die Verwendung eines derartigen Kits in der Medizin, insbesondere als Adjuvans im Rahmen einer Impfung.

Der erfindungsgemäße Extrakt zeichnet sich aufgrund seines biologischen Ursprungs durch eine geringe Toxizität und geringe Nebenwirkungen aus. Da er aus einem nachwachsenden Rohstoff herstellbar ist, ist er außerdem preisgünstig herstellbar. Wie sich aus den Beispielen ergibt, sind die Extrakte als Adjuvantien sehr wirksam und erhöhen die Resistenz gegen Infektionskrankheiten ,was zur Verringerung der Tierverluste, Reduzierung der Leistungsminderung, Erhöhung der Zuwachsraten und verbesserten Zuchtergebnissen führt.

Die erfindungsgemäßen Extrakte aus einem phototrophen Mikroorganismus weisen typischer Weise einen Gehalt an Trockenmasse zwischen 0,1 bis 6,0 g pro 100 g auf, mit einem pH-Wert von 4,0 bis 8,0, einer Leitfähigkeit von 0,3 - 13,0 mS und einem Anteil von Phaeophytinen von > 0,1 mg pro 100 g.

Die Erfindung wird nachfolgend anhand von Figuren und Beispielen weiter illustriert.

### Figuren

Es zeigen:

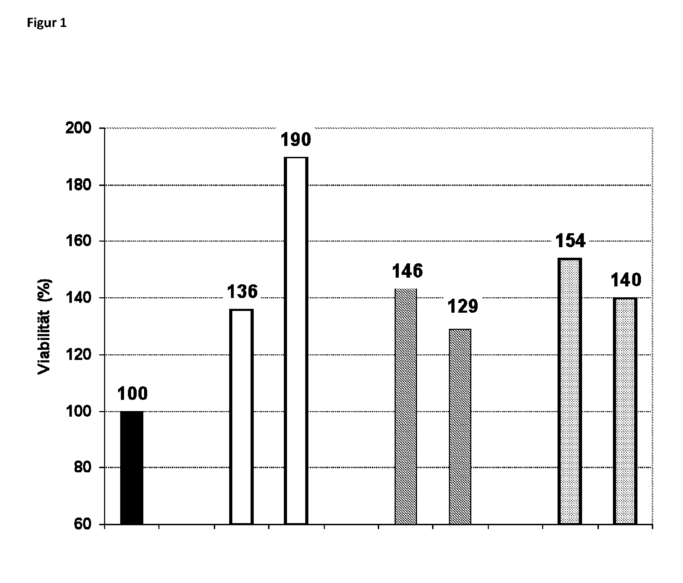

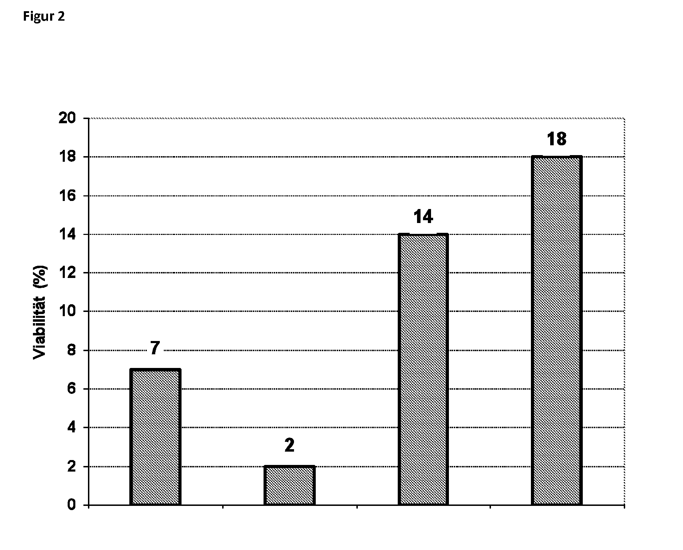

**Figuren 1** **und** **2****:** *In vitro* Prüfung der Impfstoffe mit Extrakten aus phototrophen Mikroorganismen als Adjuvantien auf die Viabilität von Zellen
   Die Charakterisierung der Adjuvantien (Extrakte) erfolgt an Schweineblut- Zellkulturen mittels eines in vitro MTT-Test. Unter definierten Bedingungen wird die Wirkung der Extrakte und der die Extrakte aufweisenden Vakzine auf die Zellaktivität und -vitalität lebender Blutzellen ermittelt.
   Gelbes Methyltetrazoliumsalz (MTT) wird durch zelluläre Dehydrogenasen zu blauem Formazan reduziert. Die Menge des gebildeten Farbstoffes korreliert mit der Zellaktivität und wird photometrisch ermittelt.
   Figur 1: *In vitro* Zellviabilität von Ferkel-Leukozyten in Abhängigkeit von Algenart, Dosierung und Präparation der Extrakte (MTT-Test)
   Figur 1 zeigt am Beispiel von Blaualgen-, Rotalgen-Extrakten und Extraktmischungen, dass die erfindungsgemäßen Adjuvantien die Zellviabilität gegenüber der Kontrolle, d. h. den Blutzellen ohne Dosierung von Adjuvantien, um bis zu 90 % steigern. Die Höhe der Steigerung der Zellaktivität ist abhängig von der Art des verwendeten phototrophen Mikroorganismus (der Algenart), der Konzentration und der Präparation der Adjuvantien. (Balken von links nach rechts: Kontrolle = unbehandelte Zellen; Blaualgenextrakt, Verdünnung 1:22; Blaualgenextrakt, Verdünnung 1:52; Rotalgenextrakt, Verdünnung 1:24; Rotalgenextrakt, diafiltriert, Verdünnung 1:42; Extrakt-Mix2 (Mischung wässriger Extrakte der Mikroalgen *Cyanidium* und *Nannochloropsis),* Verdünnung 1:52; Extrakt-Mix3 (Mischung wässriger *Nostoc-* und *Ascophyllum-* Extrakte), Verdünnung 1:12.
Figur 2: *In vitro* Zellviabilität von Ferkel- Leukozyten nach Dosierung von Impfstoffen ohne bzw. mit Öl-Adjuvans bzw. Algenextrakt-Adjuvantien (MTT-Test)
   Figur 2 zeigt ein Beispiel für einen MTT-Test mit Vakzinen, mit und ohne erfindungsgemäße Adjuvantien. Das Beispiel für das Adjuvans "Spirulina-Extrakt 1" veranschaulicht, dass die Vakzine ohne Adjuvans und mit handelsüblichem Öl-Adjuvans eine deutlich schlechtere Wirkung auf die Viabilität der Ferkel- Leukozyten hatten. Die beste Wirkung auf die Zellvialbilität wurde in diesem Beispiel mit der Dosierung von 20 % Algenextrakt-Adjuvans im Vakzin erreicht. (Balken von links nach rechts: Impfgruppe 2 ohne Adjuvans; Impfgruppe 3 Öl-Adjuvans; Impfgruppe 6 Algenextrakt = 10 %; Impfgruppe 5 Algenextrakt = 20 %).

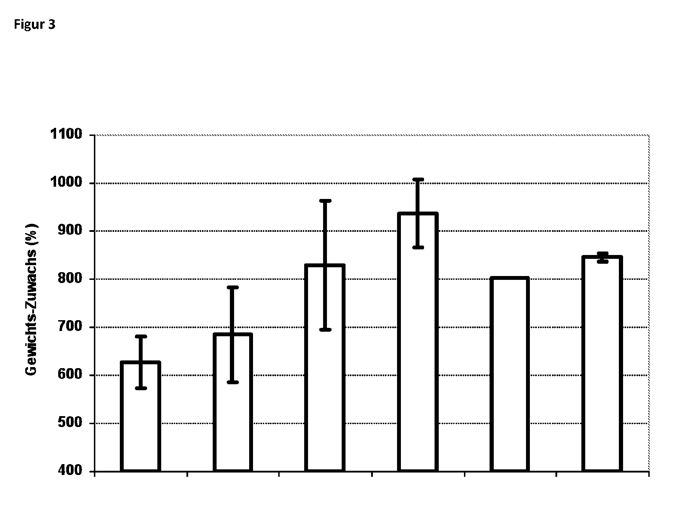

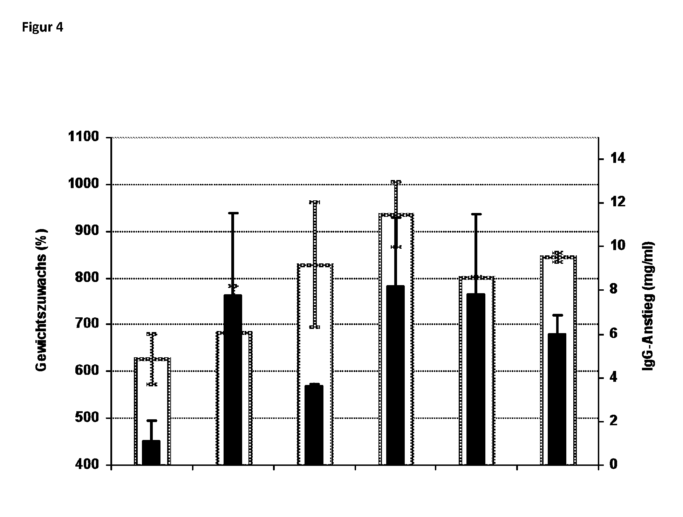

**Figuren 3** **und** **4****:** zeigen die Effekte der Impfungen mit Vakzinen, die einen Extrakt aus phototrophen Mikroorganismen (Algenextrakt) als Adjuvans enthalten, am Beispiel von *Spirulina-Extrakt* auf den Zuwachs von Ferkeln bis zur Schlachtung. Balken von links nach rechts: Kontrolle, ungeimpft; Versuchsgruppe 2, geimpft, Impfstoff ohne Adjuvans; Versuchsgruppe 3, geimpft, Impfstoff mit kommerziellem Öl-Adjuvans; Versuchsgruppe 4, geimpft, Impfstoff mit je 10 % Algen- Extrakt- und Öl- Adjuvans; Versuchsgruppe 5, geimpft, Impfstoff mit 20 % Algen- Extrakt- Adjuvans; Versuchsgruppe 6, geimpft, Impfstoff mit 10 % Algen-Extrakt-Adjuvans.
   Figur 3: Gewichtszunahme von Ferkeln bis zur Schlachtung in Prozent zum Einstallgewicht (Einstallgewicht = 100%)
   Im Vergleich zur Kontrollgruppe (ungeimpft) erreichten alle geimpften Versuchsgruppen höhere Zuwachsraten. Die beste Variante in diesem Beispiel war das Adjuvans "10 % *Spirulina*-Extrakt und 10 % Öl", also auf eine Mischung des erfindungsgemäßen Adjuvans mit Öl als herkömmlichem Adjuvans.
   Figur 4: Gewichtszunahme bis zur Schlachtung (%) und Anstieg des IgG (mg/ml) bis zum 28. Tag nach der Impfung (breite Balken: Gewichtszuwachs; schmale Balken: Ig-Anstieg).
   Figur 4 zeigt ein Beispiel für die Wirkung der Impfungen auf immunologische Parameter. Diese wurden mittels ELISA-Tests (Enzym-Linked-Imunnosorbent-Assay) mit Hilfe spezifischer, enzymgekoppelter Antikörper, die selektiv das jeweilige Protein binden (z. B. Zytokine, Immunglobuline), das über die optische Dichte quantifiziert wird, ermittelt. Es wird der Anstieg des Immunglobulin G (IgG) dargestellt. Bei allen geimpften Gruppen war bis zum 28. Tag nach der Impfung ein stärkerer Anstieg des IgGs als in der Kontrollgruppe zu beobachten. Der stärkste Anstieg wurde in der Versuchs-Gruppe "10 % *Spirulina*-Extrakt und 10 % Öl" gemessen.

### Beispiele

Die immunstimulierende Wirkung von Extrakten phototropher Mikroorganismen wurde mit bestandsspezifischen Vakzinen für Schweine, d. h. mit Impfstoffen basierend auf einem Mikroorganismenspektrum, das dem potentiellen spezifischen Krankheitsgeschehen im Tierbestand entsprach, nachgewiesen.

Die Effekte der Impfungen wurden durch *in vitro* und *in vivo* Untersuchungen z.B. mittels Zelltests, immunologische Untersuchungen und Ermittlung der Tierverluste und Wachstumsraten geprüft.

### Verwendete phototrophe Mikroorganismen

Für die Herstellung der Extrakte phototropher Mikroorganismen wurden Spezies verschiedener Algen- und Cyanobakterien- Gattungen wie *Spirulina, Chlorella, Porphyridium, Nannochloropsis, Galderia, Cyanidium , Nostoc, Scenedesmus, Kirchneriella, Dunaliella, Phormidium* eingesetzt.

### Kultivierung

Die Mikroalgen wurden unter Einhaltung der nachstehend aufgeführten Kultivierungsstufen unter speziellen kontrollierten Bedingungen im "scale up" kultiviert:
- Aktivierung des Stammes durch Überimpfen in stamm- (art-) spezifischem Flüssigmedium.
- Scale up von 10 ml über 50, 100, 500 bis zur 2.000 ml Laborkultivierung unter Steuerung von Temperatur, Bestrahlung, CO₂- Luft- Begasung und mechanische Durchmischung.
- Scale up zum kleintechnischen Maßstab 30, 100 bis zum 4.000 L- Maßstab mit on-und off-line Messung und Regelung von Temperatur, pH- Wert, optische Dichte, CO₂-Dosierung und Pumpen-Frequenz.

### Kultivierungsbedingungen:

*1. Chlorella spp.:* ½ Tamiya Medium; 20 - 35 °C; pH 7,0 - 7,5; in Abhängigkeit von der Wachstumsphase 50 ->1500 µE/(m²*s); 3% CO₂- Luftgemisch- Begasung, mit De-Immobilisierungsgranulaten bei maximaler Pumpendrehzahl.
*2. Spirulina spp.:* Zarrouk Medium; 25°C - 30°C; pH 8,0 - 10; ca. 20 - 1500 µE/(m²*s); Luft-Begasung, mit De-Immobilisierungsgranulaten bei reduzierter Pumpendrehzahl.

Die speziellen Kultivierungsbedingungen zeichnen sich im Weiteren dadurch aus, dass durch die Kultivierung eine biologische Anreicherung der Biomasse mit bestimmten Mineralstoffen wie Zn, Se, Ca u. a. erfolgt. Das wird durch Modifizierung der Nährlösung erreicht. Die Modifizierung kann die zusätzliche Dosierung von Salzen dieser Mineralien beinhalten.

Die Ernte der Algenbiomasse erfolgt durch Filtration, Separation oder Zentrifugation einschließlich Waschvorgänge. Die Waschung kann mit Wasser, physiologischer Kochsalzlösung, sauren oder alkalischen wäßrigen Lösungen erfolgen. Es kann sich eine Frostung oder Trocknung der Biomasse mittels schonender Sprüh-, Gefrier- oder Sonnentrocknung und eine Vakuumverpackung anschließen.

Im allgemeinen ist die Kultivierung von photoprophen Mikroorganismen dem Fachmann bekannt und ist u. a. in der folgenden Literatur beschrieben:
Richmond, A., et. al, 2004, Handbook of: Microalgal Culture Biotechnology and Applied Phycology, Blackwell Publishing, S.566 ff.
Iwamoto, H., 2004, Industrial Production of Microalgal Cell-mass and Secondary Products - Major Industrial Species Chlorella, In: Richmond, A., et. al, 2004. Handbook of: Microalgal Culture Biotechnology and Applied Phycology. Blackwell Publishing, 255-263.
Becker, E.W., 1994, Microalgae: Biotechnology and Microbiology, Cambridge UniversityPress, S. 301 ff.
Cohen, Z., 1999, Chemicals from Microalgae, Crc Pr Inc, 1-24, 282-291, 41-56, 261-281, 292-312.
http://www.fao.org/docrep/003/w3732e/w3732e06.htm (Webiste der "Food and Agriculture Organisation ofthe United Nations".)

### Gewinnung von Extrakten - Herstellung und Charakterisierung

Die Wirkstoffisolate werden durch Extraktionen von Biomassen gewonnen. Die Extraktion kann mit Wasser oder Ethanol-Wasser-Gemisch erfolgen. Sie beinhalten das definierte Rehydratisieren (bei definierter Konzentration, Temperatur, Dauer, Rührgeschwindigkeit), Erhitzen, Kochen, Separieren, Filtrieren, Aufkonzentrieren durch Ultra- und evt. Diafiltration, Sterilisieren und evt. Lyophilisieren. Derartige Verfahren sind dem Fachmann bekannt und werden u.a. in der folgenden Literatur beschrieben:
Molina Grima, E., et, al, Downstream Processing of Cell-mass and Products, In: Richmond, A., et. al, 2004. Handbook of: Microalgal Culture Biotechnology and Applied Phycology. Blackwell Publishing, 230-239.
Heiss, R., 2004, Lebensmitteltechnologie: Biotechnologische, chemische, mechanische und thermische Verfahren der Lebensmittelverarbeitung, Springer, Berlin, 6. Auflage, 136-138, 425-426.
Salzer, U.-J., Fred Siewek, F., 2010, Handbuch Aromen und Gewürze, Behr's Verlag Bd. 1 , 3A, 5-20.
Ziegler; E., 1982, Die natürlichen und künstlichen Aromen, Dr. Alfred Huthig Verlag, Heidelberg, 221-242.
Kingsley S. Rowan, 1989, Photosynthetic Pigments of Algae, Cambridge University Press, 192-194.

Die Herstellungstechnologie ist artspezifisch.

Die Gewinnung der Wirkstoffisolate stellt sich typischerweise folgendermaßen dar:
- 90-98 % Wasser (18-20 °C) vorlegen und 2-10 % Biomasse einrühren
- 1-14 h unter Rühren rehydratisieren
- erhitzen auf 60 °C - 98 °C
- 1-2 h unter Rühren extrahieren
- Abtrennen der groben Feststoffe durch Separation oder Zentrifugieren
- weitere Reinigung durch Mikrofiltration mit 0,45 µm Filter bei 20 °C
- Aufkonzentrieren durch Ultrafiltration, Einstellen der Trockenmassegehalte
- Stabilisieren durch Sterilfiltrieren, Autoklavieren oder Konservieren

Es werden definierte Extrakte erhalten. Das Molekulargewicht der Algenwirkstoffe in den Extrakten ist größer als 10 kDalton.

Die Extrakte werden physikalisch, chemisch, mikrobiologisch und biologisch analysiert und mit Spezifikationen und Datenblättern charakterisiert (Tabelle 1).

### Herstellung des Vakzins

Die Vakzine werden aus lebenden oder toten Antigenen, Flüssig- oder Trocken-Algenextrakten, gegebenenfalls physiologischer Kochsalzlösung, gegebenenfalls anderen Adjuvantien wie Öl oder Al(OH)₃ und weiteren Bestandteilen hergestellt.

Die Dosierung des Algenextraktes im Vakzin beträgt bis 60 %, bevorzugt bis zu 30 %, besonders bevorzugt bis zu 20 %.

**Tabelle 1: Charakterisierung der Parameter Trockenmasse, pH-Wert, Leitfähigkeit, Phaeophytine und Sterilität für einen Extrakt aus einem phototrophen Mikroorganismus (Spirulina Extrakt).**

| **Parameter** | **Methode/Gerät** | **Messwerte/Bereich** |
|---|---|---|
| Trockenmasse | DAB 10 (V.6.22.N2) Trockenmassebestimmer bei 105°C | 0,2 - 6,0 g/ 100 g |
| pH-Wert | DAB 10 (V.6.3.1) Potentiometer | 4,0 - 8,0 |
| Leitfähigkeit | Condictivitymeter FE30/ FG3 Mettler Toledo | 0,4 - 12,5 mS |
| Phaeophytine | HPLC; MALDI- TOF | >0,1 mg/ 100 g |
| | | |

| **Mikrobiologie** | | |
|---|---|---|
| Sterilität | Sterilitätskontrolle nach EU AB Ph. Euro 6.0 / 2.06.12 ; 13.00 | steril |

## Patentansprüche

1. Zusammensetzung, aufweisend:
- einen Extrakt aus einem phototrophen Mikroorganismus, und
- einen Immunstimulator zur Generierung einer aktiven Immunität.

2. Zusammensetzung nach Anspruch 1, wobei der phototrophe Mikroorganismus ausgewählt ist aus der Gruppe bestehend aus Algen und Cyanobakterien.

3. Zusammensetzung nach Anspruch 2, wobei die Algen ausgewählt sind aus der Gruppe bestehend aus Chlorophyta, Rhodophyta und Heterokontophyt.

4. Zusammensetzung nach Anspruch 1 bis 3, weiterhin aufweisend eine Substanz, die ausgewählt ist aus der Gruppe bestehend aus: Öl, Aluminiumhydroxid, komplettem Freund'schen Adjuvans, inkomplettem Freund'schen Adjuvans, stabilisierenden kationischen Peptiden ausgewählt aus Polypeptiden, Protamin, Nukleolin, Spermin oder Spermidin, kationischen Polysacchariden, Chitosan, TDM, MDP, Muramyldipeptid, sowie Alaun-Lösung, Pluronics und Lipopeptiden, einschließlich Pam3Cys.

5. Verwendung einer Zusammensetzung nach Anspruch 1 bis 4 in der Medizin, insbesondere zur Impfung.

6. Verfahren zur Herstellung einer Zusammensetzung nach Anspruch 1 bis 4, aufweisend die folgenden Schritte:
- Bereitstellen eines phototrophen Mikroorganismus,
- Herstellen eines Extraktes aus dem phototrophen Mikroorganismus und
- Mischen des Extrakts aus dem phototrophen Mikroorganismus mit einem Immunstimulator zur Generierung einer aktiven Immunität.

7. Verfahren zur Immunisierung eines Tieres gegen eine Infektionskrankheit, aufweisend den Schritt der Verabreichung einer Zusammensetzung nach Anspruch 1 bis 4.

8. Verfahren nach Anspruch 7, wobei das Tier ein Wirbeltier ist.

9. Verfahren nach Anspruch 8, wobei das Wirbeltier ein Säugetier oder ein Vogel oder ein Fisch ist.

10. Verfahren nach Anspruch 9, wobei das Säugetier ein Nutz-, Haus-, Zoo-, Wildtier oder ein Mensch ist, wobei das Haustier insbesondere ausgewählt ist aus der Gruppe bestehend aus Rind, Schwein, Pferd, Esel, Kamel, Katze, Hund, Ren, Huhn, Pute, Gans, Ente, Kaninchen, Schaf, und Ziege , und/oder wobei das Nutztier insbesondere ausgewählt ist aus der Gruppe bestehend aus Pelztieren und Fischen.

11. Verfahren nach Anspruch 7 bis 10, wobei die Infektionskrankheit eine virale oder eine bakterielle Krankheit ist, wobei die virale oder eine bakterielle Krankheit insbesondere ausgewählt ist aus der Gruppe bestehend aus:
Influenza, SARS, Gelbfieber, AIDS, Condyloma acuminata, Dellwarze, Dengue-Fieber, Dreitagefieber, Ebolavirus, Frühsommermeningoenzephalitis (FSME), Gürtelrose, Hepatitis, Herpes-Simplex Typ I, Herpes-Simplex Typ II, Herpes zoster, Japanische Enzephalitis, Lassafieber, Marburg-Virus, Masern, Maul- und Klausenseuche, Mononukleose, Mumps, Norwalk-Virus-infektion, Pfeifersches-Drüsenfieber, Pocken, Polio (Kinderlähmung), Pseudokrupp, Ringelröteln, Tollwut, Warzen, West-Nil-Fieber, Windpocken, Zytomegalie-Virus (CMV), bakteriellen Infektionskrankheiten wie Lyme-Borreliose, Anthrax, bakterielle Meningitis, Anthrax, Appendizitis (Blinddarmentzündung), Borna'sche Krankheit, Botulismus, Camphylobacter, Chlamydia trachomatis (Harnröhren-, Bindehautentzündung), Cholera, Diphterie, Donavanosis, Epiglottitis, Fleckfieber, Flecktyphys, Gasbrand, Gonorhoe, Tularämie, Heliobacter pylori, Bordetella pertussis, klimatischer Bubo, Knochenmarksentzündung, Legionärskrankheit, Lepra, Listeriose, Mittelohrentzündung, Mycoplasma hominis, Neugeborenensepsis, Noma, Paratyphus, Pest, Reitersyndrom, Rocky Mountain spotted fever, Salmonellen-Paratyphus, Salmonellen-Typhus, Scharlach, Syphilis, Tetanus, Tripper, Tuberkulose, Typhus, Vaginitis (Kolpitis), Weicher Schanker; sowie E. coli-Infektionen und Intoxikationen (E. coli-Ruhr, E.coli-Enterotoxämie, E. coli-Septikämie), Salmonelleninfektionen )insbesondere S. cholera suis, S. dublin, S. gallinarum, S. pullorum, S. enteritis, S. thyphimurium), Streptokokkeninfektionen (Druse, Streptokokken-Infektionen beim Schwein, Sudden death-Syndrom bei Enten, Streptococcus zooepidemicuns-Infektion), Staphylokokkeninfektionen (Staph. Hyicus-, Staph. Aureus-, Staph. intermedius-Infektionen), Pasteurellen-Infektionen (Pasteurella multocida-Infektionen, Geflügelcholera, Haemorrhagische Septicaemia) Wild- und Rinderseuche, Rhinitis atrophicans, Mannheimia-haemolytica-Infektion, Moraxella-Infektionen (infektiöse Keratoconjunctivitis infolge von Moraxella bovis-Infektion u. a.), Erysipelothrix rhusiopathiae-Infektion (Rotlauf, Septikämie beim Geflügel und bei Nagetieren), Moderhinke der Schafe (Bacteroides nodosus-Infektion, Fusobacterium necrophorum-Infektion), Nekrobazillose der Tiere und des Menschen (Fusobacterium necrophorumilnfektion), Clostridien-Infektionen und ilntoxikationen (Rauschbrand, Pararauschbrand, Botulismus, Tetanus, CI. perfringens-Enterotoxämie, Gasbrand, Ulzerative Enteritis der Vögel, Nekrotische Hepatitis, Bradsot/CI. Spiriformes- Infektionen, Malignes Ödem, Nekrotisierende Mastitis, Bazilläre Hämoglobinurie), Bordetellose (Bordetella bronchiseptica-Infektion, Bordetella avium-Infektion), Riemerellose (R. anatipestifer-Infektion, R. columbina-Infektion), Hämophilose (Glässersche Krankheit, Haemophilus gallinarum-Infektion), Actinobacillus, pleuropneumoniae-Infektion der Schweine, Actinobacillus equuli-Infektion der Pferde, Chlamydien-Infektionen (Enzootischer Abort der Schafe, Chlamydieninfektionen der Rinder), Ornithobacterium rhinotracheale- Infektion, Mycoplasmen-Infektionen, Arcanobacterium pyogenes-Infektion, bakterielle Infektionen bei Fischen (Furunkulose/Aeromonas salmonicida-Infektion, Rotmaulkrankheit/ Yersinia ruckeri-Infektion, Vibriosen (Vibrio anguillarum-Infektion)).

12. Verwendung eines Extrakts aus einem phototrophen Mikroorganismus in der Medizin, insbesondere als Adjuvans.

13. Verwendung nach Anspruch 12, wobei der phototrophe Mikroorganismus ausgewählt ist aus der Gruppe bestehend aus Algen und Cyanobakterien.

14. Verwendung nach Anspruch 13, wobei die Algen ausgewählt sind aus der Gruppe bestehend aus Chlorophyta, Rhodophyta und Heterokontophyta.

15. Kit, aufweisend
- ein wässrigen Extrakt aus einem phototrophen Mikroorganismus und
- einen Immunstimulator zur Generierung einer aktiven Immunität.

16. Verwendung eines Kits nach Anspruch 15 in der Medizin, insbesondere als Adjuvans.
